# EUROPÄISCHE PATENTANMELDUNG

(11) **EP 2 230 237 A1**
(43) Veröffentlichungstag der Anmeldung: **22.09.2010**
(21) Anmeldenummer: 09155202.6
(22) Anmeldetag: 16.03.2009
(51) Int. Cl.: C07D 405/12

(54) **Neues Verfahren zur Herstellung von Enaminocarbonyl-Verbindungen**

(71) Anmelder: Bayer CropScience AG, 40789 Monheim (DE)
(72) Erfinder: Die Erfindernennung liegt noch nicht vor

(57) **Zusammenfassung**

Die vorliegende Erfindung betrifft ein Verfahren zur Herstellung von Enaminocarbonylverbindungen der Formel (I) 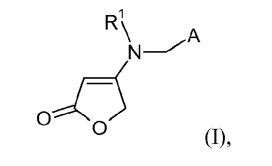 wobei Verbindungen der Formel (II) 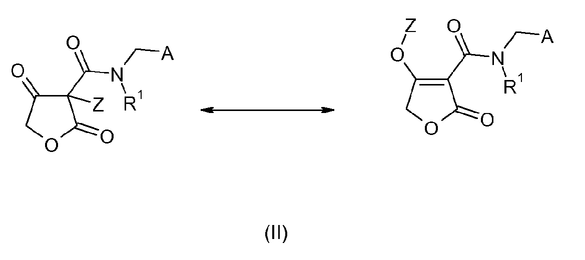 zu Verbindungen der Formel (I) umgesetzt werden und A, R1 und Z wie in der Beschreibung definiert sind, sowie entsprechende Ausgangsverbindungen, welche in dem erfindungsgemäßen Verfahren verwendet werden.

## Beschreibung

Die vorliegende Erfindung betrifft ein Verfahren zur Herstellung von 4-Amino-but-2-enoliden.

Bestimmte substituierte Enaminocarbonyl-Verbindungen sind als insektizid wirksame Verbindungen aus der EP 0 539 588 A1 bekannt. Darüber hinaus beschreiben auch die internationalen Patentanmeldungen WO 2007/115644, WO 2007/115643 und WO 2007/115646 entsprechende insektizid wirksame Enaminocarbonyl-Verbindungen.

Im Allgemeinen werden Enaminocarbonylverbindungen aus Tetronsäure und einem Amin gemäß dem nachfolgenden Schema 1 synthetisiert. Diese Vorgehensweise ist beispielsweise in EP 0 539 588 A1 sowie in Heterocycles Vol. 27, Nr. 8, Seite 1907 bis 1923 (1988) beschrieben.

### Schema 1:

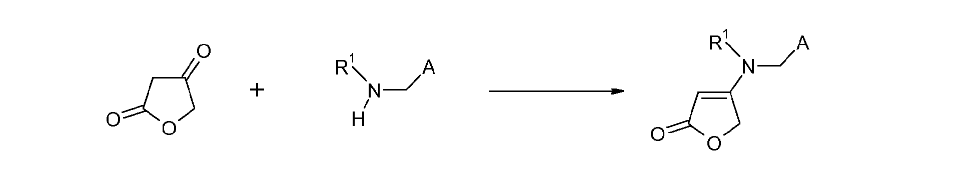

Nachteilig an diesem Verfahren ist insbesondere, dass man wasserfreie Tetronsäure als Ausgangsverbindung benötigt, deren Herstellung aufwendig und kostenintensiv ist.

So wird Tetronsäure im Allgemeinen ausgehend von Acetessigester über eine Bromierung und anschließende Hydrierung hergestellt (vg1. Synthetic Communication, 11(5), Seiten 385 bis 390 (1981)). Die Gesamtausbeute an Tetronsäure ausgehend von Acetessigester ist dabei kleiner als 40 %, was das Verfahren unter industriellem Gesichtspunkt wenig attraktiv macht.

In der CH-PS 503 722 ist ein weiteres Verfahren zur Herstellung von Tetronsäure beschrieben.
Dabei wird 4-Chloracetessigester mit einem aromatischen Amin zum 3-Arylaminocrotonlacton umgesetzt und anschließend wird die Tetronsäure durch Behandlung mit Mineralsäuren freigesetzt.
Der Nachteil an diesem Verfahren besteht darin, dass die Isolierung der Tetronsäure nur durch Hochvakuum-Sublimation möglich ist, was auch dieses Verfahren unter industriellem Gesichtspunkt wenig attraktiv macht.

Ein weiteres Verfahren zur Herstellung von Tetronsäure ist in der EP 0 153 615 A beschrieben, in welchem von 2,4-Dichloracetessigestern ausgegangen wird. Dieses ebenfalls mehrstufige und aufwändige Verfahren liefert die gewünschte Verbindung ebenfalls nur mit einer mäßigen Gesamtausbeute von 65 %.

In Tetrahedron Letters, Nr. 31, Seiten 2683 und 2684 (1974) ist die Herstellung von Tetronsäure und einer entsprechenden Enaminocarbonylverbindung beschrieben. Die dort beschriebene Synthese ist in folgendem Schema 2 wiedergegeben. Als Edukt wird dabei Acetylendicarbonsäuredimethylester eingesetzt.

### Schema 2:

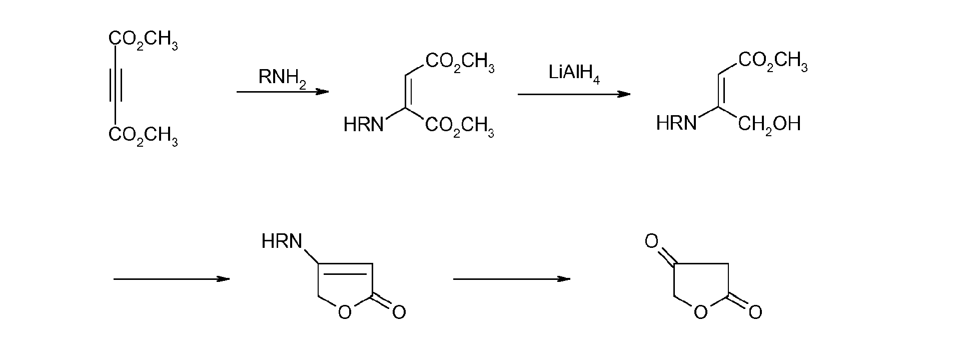

Nachteilig an diesem Verfahren ist die geringe Gesamtausbeute von nur 30 % sowie das Erfordernis, kostenintensive Edukte, beispielsweise Lithiumaluminiumhydrid (LiAlH₄), als Reagenzien verwenden zu müssen.

Ferner ist aus dem Stand der Technik ein Verfahren zur Herstellung von Enaminocarbonyl-Verbindungen ausgehend von Methyltetronat bekannt (J. Heterocyclic Chem., 21, 1753 (1984)). Für dieses Verfahren wird als Ausgangsmaterial der kostenintensive 4-Brom-3-methoxy-but-3-encarbonsäureester verwendet.

Ein weiteres Verfahren geht von einem 4-Chloracetessigester aus, der mit Aminen umgesetzt wird (Heterocycles, Vol. 27, Nr. 8, 1988, Seiten 1907 bis 1923). Die Reaktion zum Aminofuran wird in einem Schritt durchgeführt. Dabei wird das Amin mit Eisessig zu einer Lösung von 4-Chloracetessigester in Benzol gegeben und die resultierende Mischung wird mehrere Stunden unter Rückfluss erhitzt. Die Ausbeuten an 4-Methylamino-2(5H)-furanon in dieser Synthese betragen nur 40%.

Aus EP 0 123 095 A ist ein Verfahren bekannt, in welchem Tetronsäureamid aus 3-Amino-4-acetoxycrotonsäureester hergestellt wird. 3-Amino-4-acetoxycrotonsäureester ist kostenintensiv und aufwändig herzustellen, so dass eine wirtschaftliche Synthese mit diesem Verfahren nicht möglich ist.

Ein weiteres Verfahren zur Herstellung von Tetronsäure ausgehend von Malonestern und Chloracetylchlorid ist aus J. Chem. Soc., Perkin Trans. 1 (1972), Nr. 9/10, Seiten 1225 bis 1231 bekannt. Dieses Verfahren liefert die gewünschte Zielverbindung mit einer Ausbeute von nur 43 %.

In der vorstehend erwähnten, Internationalen Patentanmeldung WO 2007/115644 wird die Herstellung von Enaminocarbonyl-Verbindungen, beispielsweise von 4-[[(6-Chlorpyridin-3-yl)methyl](3,3-dichlorprop-2-en-1-yl)amino]furan-2(5H)-on durch Umsetzung von 4-[[(6-Chlorpyridin-3-yl)methyl]amino]furan-2(5H)-on mit 3-Brom-1,1-dichlorprop-1-en, beschrieben (vgl. Herstellungsbeispiel, Verfahren 2, Beispiel (3)). Die WO 2007/115644 beschreibt auch die Herstellung von Enaminocarbonyl-Verbindungen, beispielsweise von 4-[[(6-Chlorpyridin-3-yl)methyl](2-Fluorethyl)amino]furan-2(5H)-on durch Umsetzung von 4-[[(2-Fluorethyl)amino]furan-2(5H)-on mit 2-Chlor-5-chlormethyl-pyridin (vgl. Herstellungsbeispiele, Verfahren 3, Beispiel (4)). Die Reaktionen werden vorzugsweise mit Hydriden des Lithiums oder Natriums durchgeführt. Diese Substrate sind im Allgemein kostenintensiv und gleichzeitig aus Sicherheitsgründen nur schwer zu handhaben.

In der nicht vorveröffentlichten EP 07116639 werden Enaminocarbonylverbindungen beispielsweise ausgehend von 4-(Methoxycarbonyl)-5-oxo-2,5-dihydrofuran-3-ol und einem Amin hergestellt.

### Schema 3:

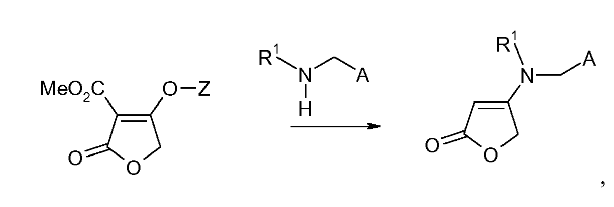

wobei
R¹ für Wasserstoff, Alkyl, Halogenalkyl, Alkenyl, Halogenalkenyl, Alkinyl, Cycloalkyl, Cycloalkylalkyl, Halogencycloalkyl, Alkoxy, Alkyloxyalkyl, Halogencyclo-alkylalkyl oder Arylalkyl steht;
Z für Wasserstoff, Alkalimetall oder Erdalkalimetall steht; und
A Pyrid-2-yl oder Pyrid-4-yl steht oder für Pyrid-3-yl, welches gegebenenfalls in 6-Position substituiert ist durch Fluor, Chlor, Brom, Methyl, Trifluormethyl oder Trifluormethoxy oder für Pyridazin-3-yl, welches gegebenenfalls in 6-Position substituiert ist durch Chlor oder Methyl oder für Pyrazin-3-yl oder für 2-Chlor-pyrazin-5-yl oder für 1,3-Thiazol-5-yl, welches gegebenenfalls in 2-Position substituiert ist durch Chlor oder Methyl, oder für
Pyrimidinyl, Pyrazolyl, Thiophenyl, Oxazolyl, Isoxazolyl, 1,2,4-Oxadiazolyl, Isothiazolyl, 1,2,4-Triazolyl oder 1,2,5-Thiadiazolyl steht, welcher gegebenenfalls durch Fluor, Chlor, Brom, Cyano, Nitro, C₁-C₄-Alkyl (welches gegebenenfalls durch Fluor und/oder Chlor substituiert ist), C₁-C₃-Alkylthio (welches gegebenenfalls durch Fluor und/oder Chlor substituiert ist), oder C₁-C₃-Alkylsulfonyl (welches gegebenenfalls durch Fluor und/oder Chlor substituiert ist), substituiert ist, oder für 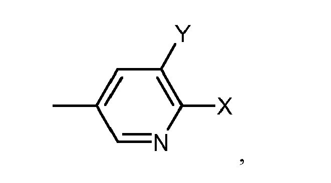 in welchem
X für Halogen, Alkyl oder Halogenalkyl steht und
Y für Halogen, Alkyl, Halogenalkyl, Halogenalkoxy, Azido oder Cyan steht.
Ausgehend von diesem Stand der Technik ergibt sich als Aufgabe der vorliegenden Erfindung, ein alternatives Verfahren zur Herstellung von Enaminocarbonyl-Verbindungen bereitzustellen, welches vorzugsweise einfach und kostengünstig durchzuführen ist. Die mit diesem angestrebten Verfahren erhältlichen Enaminocarbonyl-Verbindungen sollen dabei vorzugsweise mit hoher Ausbeute und hoher Reinheit erhalten werden. Insbesondere soll das angestrebte Verfahren den Erhalt der gewünschten Zielverbindungen ohne die Notwendigkeit komplexer Aufreinigungsmethoden ermöglichen.

Gelöst wird diese Aufgabe durch ein neues Verfahren zur Herstellung von Enaminocarbonyl-Verbindungen der allgemeinen Formel (I):

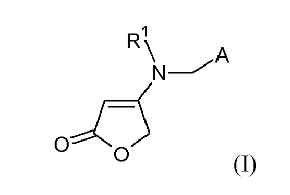

Das erfindungsgemäße Verfahren ist **dadurch gekennzeichnet, dass** man Verbindungen der allgemeinen Formel (II) 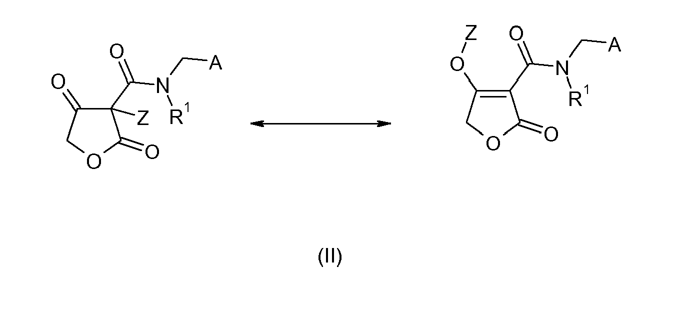 zu Verbindungen der Formel (I) umsetzt, wobei
R¹ für Wasserstoff, Alkyl, Halogenalkyl, Alkenyl, Halogenalkenyl, Alkinyl, Cycloalkyl, Cycloalkylalkyl, Halogencycloalkyl, Alkoxy, Alkyloxyalkyl, Halogencycloalkylalkyl, Aryl oder Arylalkyl steht;
Z für Wasserstoff, Alkalimetall oder Erdalkalimetall steht; und
A für Pyrid-2-yl oder Pyrid-4-yl steht oder für Pyrid-3-yl, welches gegebenenfalls in 6-Position substituiert ist durch Fluor, Chlor, Brom, Methyl, Trifluormethyl oder Trifluormethoxy oder für Pyridazin-3-yl, welches gegebenenfalls in 6-Position substituiert ist durch Chlor oder Methyl oder für Pyrazin-3-yl oder für 2-Chlor-pyrazin-5-yl oder für 1,3-Thiazol-5-yl, welches gegebenenfalls in 2-Position substituiert ist durch Chlor oder Methyl, oder für
Pyrimidinyl, Pyrazolyl, Thiophenyl, Oxazolyl, Isoxazolyl, 1,2,4-Oxadiazolyl, Isothiazolyl, 1,2,4-Triazolyl oder 1,2,5-Thiadiazolyl steht, welcher gegebenenfalls durch Fluor, Chlor, Brom, Cyano, Nitro, C₁-C₄-Alkyl (welches gegebenenfalls durch Fluor und/oder Chlor substituiert ist), C₁-C₃-Alkylthio (welches gegebenenfalls durch Fluor und/oder Chlor substituiert ist), oder C₁-C₃-Alkylsulfonyl (welches gegebenenfalls durch Fluor und/oder Chlor substituiert ist), substituiert ist, oder für 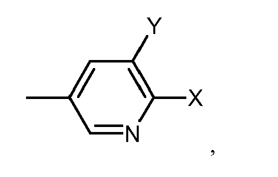 in welchem
X für Halogen, Alkyl oder Halogenalkyl steht und
Y für Halogen, Alkyl, Halogenalkyl, Halogenalkoxy, Azido oder Cyan steht.

Erfindungsgemäß ist somit vorgesehen, dass die gewünschten Enaminocarbonyl-Verbindungen der allgemeinen Formel (I) durch eine Umsetzung der entsprechenden Verbindungen der allgemeinen Formel (II) hergestellt werden. Die gewünschten Enaminocarbonyl-Verbindungen der allgemeinen Formel (I) werden unter den erfindungsgemäßen und weiter unten näher spezifizierten bevorzugten Reaktionsbedingungen mit guten Ausbeuten in hoher Reinheit erhalten, womit das erfindungsgemäße Verfahren die oben genannten Nachteile der Verfahren des Standes der Technik überwindet. Die gewünschten Verbindungen werden dabei in einer Reinheit erhalten, welche eine umfangreiche Aufarbeitung des unmittelbaren Reaktionsprodukts im Allgemeinen nicht erforderlich macht.

Bevorzugte, besonders bevorzugte und ganz besonders bevorzugte Bedeutungen des in den oben erwähnten allgemeinen Formeln (I) und (II) aufgeführten Restes A und R¹ werden im Folgenden erläutert.

A wird bevorzugt ausgewählt aus der Gruppe bestehend aus 6-Fluor-pyrid-3-yl, 6-Chlor-pyrid-3-yl, 6-Brom-pyrid-3-yl, 6-Methyl-pyrid-3-yl, 6-Trifluormethyl-pyrid-3-yl, 6-Trifluormethoxypyrid-3-yl, 6-Chlor-1,4-pyridazin-3-yl, 6-Methyl-1,4-pyridazin-3-yl, 2-Chlor-1,3-thiazol-5-yl oder 2-Methyl-1,3-thiazol-5-yl, 2-Chlor-pyrimidin-5-yl, 2-Trifluormethyl-pyrimidin-5-yl, 5,6-Difluor-pyrid-3-yl, 5-Chlor-6-fluor-pyrid-3-yl, 5-Brom-6-fluor-pyrid-3-yl, 5-Iod-6-fluor-pyrid-3-yl, 5-Fluor-6-chlor-pyrid-3-yl, 5,6-dichlor-pyrid-3-yl, 5-Brom-6-chlor-pyrid-3-yl, 5-Iod-6-chlor-pyrid-3-yl, 5-Fluor-6-brom-pyrid-3-yl, 5-Chlor-6-brom-pyrid-3-yl, 5,6-Dibrom-pyrid-3-yl, 5-Fluor-6-iod-pyrid-3-yl, 5-Chlor-6-iod-pyrid-3-yl, 5-Brom-6-iod-pyrid-3-yl, 5-Methyl-6-fluor-pyrid-3-yl, 5-Methyl-6-chlor-pyrid-3-yl, 5-Methyl-6-brom-pyrid-3-yl, 5-Methyl-6-iod-pyrid-3-yl, 5-Difluormethyl-6-fluor-pyrid-3-yl, 5-Difluormethyl-6-chlor-pyrid-3-yl, 5-Difluormethyl-6-brom-pyrid-3-yl und 5-Difluormethyl-6-iod-pyrid-3-yl.

R¹ wird bevorzugt ausgewählt aus Wasserstoff, Alkyl, Halogenalkyl, Alkenyl, Halogenalkenyl, Alkinyl, Cycloalkyl, Cycloalkylalkyl, Halogencycloalkyl, Halogencycloalkylalkyl und Alkoxyalkyl.

Z wird bevorzugt ausgewählt aus der Gruppe bestehend aus Alkalimetallen und Wasserstoff;

A wird besonders bevorzugt ausgewählt aus der Gruppe, bestehend aus 6-Fluor-pyrid-3-yl, 6-Chlor-pyrid-3-yl, 6-Brompyrid-3-yl, 6-Chlor-1,4-pyridazin-3-yl, 2-Chlor-1,3-thiazol-5-yl, 2-Chlor-pyrimidin-5-yl, 5-Fluor-6-chlor-pyrid-3-yl, 5,6-Dichlor-pyrid-3-yl, 5-Brom-6-chlor-pyrid-3-yl, 5-Fluor-6-brom-pyrid-3-yl, 5-Chlor-6-brom-pyrid-3-yl, 5,6-Dibrom-pyrid-3-yl, 5-Methyl-6-chlor-pyrid-3-yl, 5-Chlor-6-iod-pyrid-3-yl und 5-Difluormethyl-6-chlor-pyrid-3-yl.

R¹ wird besonders bevorzugt ausgewählt aus der Gruppe bestehend aus Methyl, Ethyl, Propyl, Vinyl, Allyl, Propargyl, Cyclopropyl, Alkoxyalkyl, 2-Fluor-ethyl, 2,2-Difluor-ethyl und 2-Fluor-cyclopropyl.

Z wird besonders bevorzugt ausgewählt aus der Gruppe bestehend aus Wasserstoff, Natrium und Kalium;

A wird ganz besonders bevorzugt ausgewählt aus der Gruppe bestehend aus 6-Chlor-pyrid-3-yl, 6-Brom-pyrid-3-yl, 6-Chlor-1,4-pyridazin-3-yl, 2-Chlor-1,3-thiazol-5-yl, 5-Fluor-6-chlor-pyrid-3-yl und 5-Fluor-6-brom-pyrid-3-yl.

R¹ wird ganz besonders bevorzugt ausgewählt aus der Gruppe bestehend aus Methyl, Ethyl, n-Propyl, n-Prop-2-enyl, n-Prop-2-inyl, Cyclopropyl, Methoxyethyl, 2-Fluorethyl und 2,2-Difluor-ethyl.

Z wird ganz besonders bevorzugt ausgewählt aus der Gruppe bestehend aus Natrium und Wasserstoff;

In einer bevorzugten Ausführungsform der vorliegenden Erfindung werden in dem erfindungsgemäßen Verfahren Ausgangsverbindungen der allgemeinen Formel (II) eingesetzt, in welchen die Substituenten A, Z und R¹ die vorstehend erwähnten bevorzugten Bedeutungen aufweisen.

In einer besonders bevorzugten Ausführungsform der vorliegenden Erfindung werden in dem erfindungsgemäßen Verfahren Ausgangsverbindungen der allgemeinen Formel (II) eingesetzt, in welchen die Substituenten A, Z und R¹ die vorstehend erwähnten besonders bevorzugten Bedeutungen aufweisen.

In einer ganz besonders bevorzugten Ausführungsform der vorliegenden Erfindung werden in dem erfindungsgemäßen Verfahren Ausgangsverbindungen der allgemeinen Formel (II) eingesetzt, in welchen die Substituenten A, Z und R¹ die vorstehend erwähnten ganz besonders bevorzugten Bedeutungen aufweisen.

Im Rahmen der vorliegenden Erfindung wird - unabhängig von den einzelnen oben erwähnten bevorzugten, besonders bevorzugten und ganz besonders bevorzugten Bedeutungen - den einzelnen verwendeten Resten im Allgemeinen folgende Bedeutung zugewiesen:

Unter dem Begriff "Alkyl", entweder in Alleinstellung oder aber in Kombination mit weiteren Begriffen, wie beispielsweise Halogenalkyl, Alkoxyalkyl, Cycloalkylalkyl, Halogencycloalkylalkyl und Arylalkyl, wird im Rahmen der vorliegenden Erfindung ein Rest einer gesättigten, aliphatischen Kohlenwasserstoffgruppe mit 1 bis 12 Kohlenstoffatomen verstanden, die verzweigt oder unverzweigt sein kann. Beispiele für C₁-C₁₂-Alkylreste sind Methyl, Ethyl, n-Propyl, iso-Propyl, n-Butyl, iso-Butyl, sek.-Butyl, tert.-Butyl, n-Pentyl, iso-Pentyl, Neopentyl, tert.-Pentyl, 1-Methylbutyl, 2-Methylbutyl, 1-Ethylpropyl, 1,2-Dimethylpropyl, Hexyl, n-Heptyl, n-Octyl, n-Nonyl, n-Decyl, n-Undecyl und n-Dodecyl. Von diesen Alkylresten sind C₁-C₆-Alkylreste besonders bevorzugt. Insbesondere bevorzugt sind C₁-C₄-Alkylreste, speziell Methyl und Ethyl.

Unter dem Begriff "Alkenyl" wird erfindungsgemäß ein linearer oder verzweigter C₁-C₁₂-Alkenylrest, welcher mindestens eine Doppelbindung aufweist, beispielsweise Vinyl, Allyl, 1-Propenyl, Isopropenyl, 1-Butenyl, 2-Butenyl, 3-Butenyl, 1,3-Butadienyl, 1-Pentenyl, 2-Pentenyl, 3-Pentenyl, 4-Pentenyl, 1,3-Pentadienyl, 1-Hexenyl, 2-Hexenyl, 3-Hexenyl, 4-Hexenyl, 5-Hexenyl und 1,4-Hexadienyl, verstanden. Bevorzugt hiervon sind C₂-C₆-Alkenylreste und besonders bevorzugt sind C₂-C₄-Alkenylreste.

Unter dem Begriff "Alkinyl" wird erfindungsgemäß ein linearer oder verzweigter C₃-C₁₂-Alkinylrest, welcher mindestens eine Dreifachbindung aufweist, beispielsweise Ethinyl, 1-Propinyl und Propargyl, verstanden. Bevorzugt hiervon sind C₃-C₆-Alkinylreste und besonders bevorzugt sind C₃-C₄-Alkinylreste. Der Alkinylrest kann dabei auch mindestens eine Doppelbindung aufweisen.

Unter dem Begriff "Cycloalkl" wird erfindungsgemäß ein C₃-C₈-Cycloalkylrest verstanden, beispielsweise Cyclopropyl, Cyclobutyl, Cyclopentyl, Cyclohexyl, Cycloheptyl und Cyclooctyl, verstanden. Bevorzugt hiervon sind C₃-C₆-Cycloalkylreste.

Unter dem Begriff "Aryl" wird erfindungsgemäß ein aromatischer Rest mit 6 bis 14 Kohlenstoffatomen, vorzugsweise Phenyl, verstanden.

Unter dem Begriff "Arylalkyl" wird eine Kombination von erfindungsgemäß definierten Resten "Aryl" und "Alkyl" verstanden, wobei der Rest im Allgemeinen über die Alkylgrupe gebunden wird, Beispiele hierfür sind Benzyl, Phenylethyl oder α-Methylbenzyl, wobei Benzyl besonders bevorzugt ist.

Im Rahmen der vorliegenden Erfindung werden unter dem Begriff "durch Halogen substituierte Reste", beispielsweise Halogenalkyl, einfach oder mehrfach bis zur maximal möglichen Substituentenzahl halogenierte Reste verstanden. Bei mehrfacher Halogenierung können die Halogenatome gleich oder verschieden sein. Halogen steht dabei für Fluor, Chlor, Brom oder Iod, insbesondere für Fluor, Chlor oder Brom.

Unter dem Begriff "Alkoxy", entweder in Alleinstellung oder aber in Kombination mit weiteren Begriffen, wie beispielsweise Halogenalkoxy, wird vorliegend ein Rest O-Alkyl verstanden, wobei der Begriff "Alkyl" die oben stehende Bedeutung aufweist.

Gegebenenfalls substituierte Reste können einfach oder mehrfach substituiert sein, wobei bei einer Mehrfachsubstitution die Substituenten gleich oder verschieden sein können.

Ein weiterer Gegenstand der vorliegenden Erfindung sind Verbindungen der Formel (II) 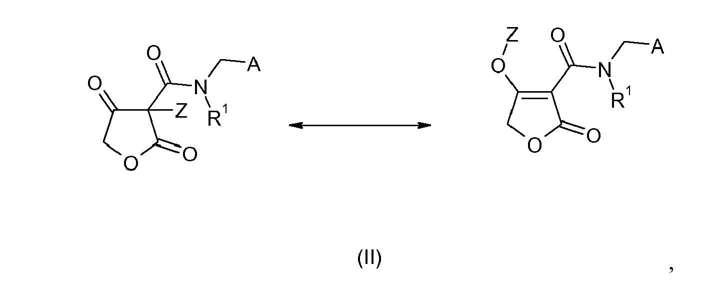 in welcher die Reste A, Z und R¹ wie oben definiert sind.

Die Synthese entsprechender abgewandelter Derivate der Verbindungen der allgemeinen Formel (II) kann gemäß nachfolgendem Schema 4 beispielsweise ausgehend von 2,4-Dioxotetrahydrofuran-3-carboxylaten der allgemeinen Formel (IV) mit Aminen der Formel (III) durchgeführt werden oder nach Literatur hergestellt werden (Bertolini et al., J. Med. Chem. 1997, 40, 2011 - 2016, Benary, Ber., 1908, 41, 1943.):

### Schema 4:

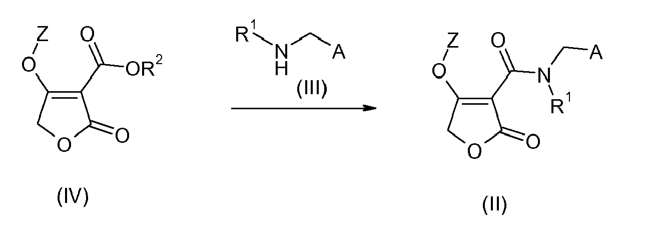

wobei die Reste A, Z und R¹ wie oben definiert sind und R² für Alkyl, Aryl oder Arylalkyl steht.

Die Verbindung (II) kann auch in einer Isomerenform vorliegen.

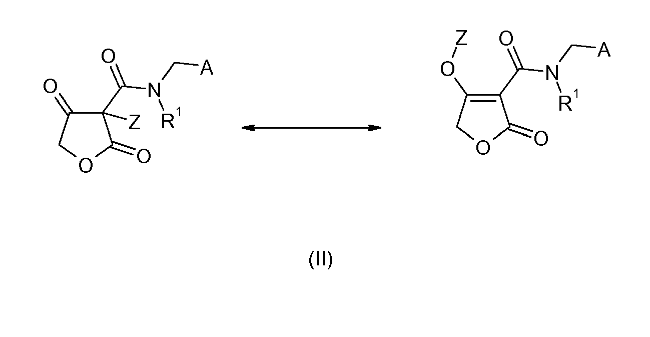

Die als Edukte verwendeten 2,4-Dioxotetrahydrofuran-3-carboxylate der allgemeinen Formel (IV) können nach aus dem Stand der Technik bekannten Verfahren hergestellt werden (R. Anschütz, Ber., 1912, 45, 2374; E. Benary, Ber., 1912, 45, 3682). Die Amine der allgemeinen Formel (III) sind kommerziell erhältlich oder können nach literaturbekannten Verfahren hergestellt werden (vgl. z.B. S. Patai "The Chemistry of Amino Group", Interscience Publishers, New York, 1968).

Die oben dargestellte erfindungsgemäße Umsetzung der Verbindungen der allgemeinen Formel (II) zu Verbindungen der allgemeinen Formel (I) wird in Gegenwart von Lösungsmitteln (Verdünnungsmitteln) durchgeführt. Lösungsmittel werden vorteilhaft in einer solchen Menge eingesetzt, dass das Reaktionsgemisch während des ganzen Verfahrens gut rührbar bleibt. Als Lösungsmittel zur Durchführung des erfindungsgemäßen Verfahrens kommen alle unter den Reaktionsbedingungen inerten organischen Lösungsmittel in Frage.

Als Beispiele sind zu nennen: Halogenkohlenwasserstoffe, insbesondere Chlorkohlenwasserstoffe, wie Tetrachlorethylen, Tetrachlorethan, Dichlorpropan, Methylenchlorid, Dichlorbutan, Chloroform, Tetrachlorkohlenstoff, Trichlorethan, Trichlorethylen, Pentachlorethan, Difluorbenzol, 1,2-Dichlorethan, Chlorbenzol, Brombenzol, Dichlorbenzol, Chlortoluol, Trichlorbenzol; Ether, wie Ethylpropylether, Methyl-tert-butylether, *n*-Butylether, Anisol, Phenetol, Cyclohexylmethylether, Dimethylether, Diethylether, Dimethylglycol, Diphenylether, Dipropylether, Diisopropylether, Di-*n-*butylether, Diisobutylether, Diisoamylether, Ethylenglycoldimethylether, Isopropylethylether, Methyl-tert-butylether, Tetrahydrofuran, Methyl-Tetrahydrofuran, Dioxan, Dichlordiethylether; Methyl-THF und Polyether des Ethylenoxids und/oder Propylenoxids; Nitrokohlenwasserstoffe wie Nitromethan, Nitroethan, Nitropropan, Nitrobenzol, Chlornitrobenzol, o-Nitrotoluol; Nitrile wie Acetonitril, Methylnitril, Propionitril, Butyronitril, Isobutyronitril, Benzonitril, Phenylnitril, m-Chlorbenzonitril sowie Verbindungen wie Tetrahydrothiophendioxid und Dimethylsulfoxid, Tetramethylensulfoxid, Dipropylsulfoxid, Benzylmethylsulfoxid, Düsobutylsulfoxid, Dibutylsulfoxid, Diisoamylsulfoxid; Sulfone wie Dimethyl-, Diethyl-, Dipropyl-, Dibutyl-, Diphenyl-, Dihexyl-, Methylethyl-, Ethylpropyl-, Ethylisobutyl- und Pentamethylensulfon; aliphatische, cycloaliphatische oder aromatische Kohlenwasserstoffe wie Pentan, n-Hexan, n-Heptan, n-Oktan, Nonan beispielsweise sogenannte White Spirits mit Komponenten mit Siedepunkten im Bereich beispielsweise von 40°C bis 250°C, Cymol, Benzinfraktionen innerhalb eines Siedeintervalles von 70°C bis 190°C, Cyclohexan, Methylcyclohexan, Petrolether, Ligroin, Octan, Benzol, Toluol, Xylol; Ester wie Methyl-, Ethyl-, Butyl-, Isobutylacetat, sowie Dimethyl-, Dibutyl-, Ethylencarbonat; Amide wie Hexamethylenphosphorsäuretriamid, Formamid, N,N-Dimethyl-acetamid, *N*-Methylformamid, *N*,*N*-Dimethyl-formamid, *N,N*-Dipropyl-formamid, *N,N*-Dibutyl-formamid, *N*-Methylpyrrolidin, *N*-Methyl-caprolactam, 1,3-Dimethyl-3,4,5,6-tetrahydro-2(1H)-pyrimidin, Octylpyrrolidon, Octylcaprolactam, 1,3-Dimethyl-2-imidazolindion, *N*-Formyl-piperidin, *N,N'*-1,4-Diformyl-piperazin; und aliphatische Alkohole, wie Methanol, Ethanol, n-Propanol und iso-Propanol und n-Butanol.

Die erfindungsgemäße Umsetzung wird vorzugsweise in einem Lösungsmittel durchgeführt, welches ausgewählt ist aus der Gruppe, bestehend aus Dioxan, Butyronitril, Propionitril, Acetonitril, DME, Toluol, Methyl-THF, Dichlorbenzol, Chlorbenzol, n-Heptan, iso-Butanol, n-Butanol, Ethanol, Methyl-tert.-butylether, Isopropylethylether und Mischungen davon.

Die Reaktion kann auch in Gegenwart von Wasser durchgeführt werden.

Die Umsetzung der Verbindungen der allgemeinen Formel (II) wird vorzugsweise in Gegenwart einer Brønsted-Säure durchgeführt.

Dabei ist es möglich, sowohl organische als auch anorganische Säuren zu verwenden. Vorzugsweise werden anorganische Säuren, beispielsweise Phosphorsäure (H₃PO₄), Schwefelsäure (H₂SO₄), Salzsäure (HCl), Bromwasserstoffsäure (HBr), Flusssäure (HF) oder Kaliumhydrogensulfat (KHSO₄), verwendet. Die einzelnen Säuren können dabei sowohl in wasserfreier Form als auch in wasserhaltiger Form, beispielsweise als 85%ige Phosphorsäure oder 37%ige Salzsäure, d.h. insbesondere in Formen, in welchen die Säuren kommerziell erhältlich sind, verwendet werden. Beispiele für geeignete organische Säuren sind Trifluoressigsäure, Essigsäure, Methansulfonsäure und p-Toluolsulfonsäure. Von den zuvor genannten Säuren sind insbesondere Phosphorsäure, Schwefelsäure, Kaliumhydrogensulfat und Trifluoressigsäure bevorzugt.

Die Umsetzung zur Herstellung der Verbindungen der allgemeinen Formel (I) kann im Allgemeinen im Vakuum, bei Normaldruck oder unter Überdruck durchgeführt werden. Die angewendeten Temperaturen können ebenfalls, in Abhängigkeit der verwendeten Substrate, variieren und sind für den Fachmann durch Routineversuche leicht zu ermitteln. Beispielsweise kann die Umsetzung zur Herstellung der Verbindungen der allgemeinen Formel (I) bei einer Temperatur von 20 bis 200 °C, vorzugsweise 20 bis 150 °C, durchgeführt werden.

Zum Ende der Reaktion kann das Reaktionswasser durch Destillation eines Teils des Lösungsmittel als Azeotrop entfernt werden. Bei hochsiedenden Lösungsmittel kann dies im Vakuum erfolgen. Durch diesen Vorgang erreicht man im Allgemeinen einen quantitativen Umsatz.

Falls die Umsetzung in einem Lösungsmittel durchgeführt wird, kann das Lösungsmittel nach dem Reaktionsende durch Abdestillieren entfernt werden. Dieses kann unter Normaldruck oder erniedrigtem Druck bei Raumtemperatur oder erhöhten Temperaturen erfolgen.

Die Isolierung der gewünschten Verbindungen der allgemeinen Formel (I) kann beispielsweise auch durch Kristallisation erfolgen.

Die vorliegende Erfindung wird anhand der nachfolgenden Beispiele näher erläutert, wobei die Beispiele nicht in die Erfindung einschränkende Weise zu interpretieren sind.

### Herstellungsbeispiele:

### Beispiel 1 Herstellung von 4-[[(6-Chlorpyridin-3-yl)methyl](2,2-difluorethyl)amino]furan-2(5H)-on

Zu einer Suspension von 1,7 g N-[(6-Chlorpyridin-3-yl)methyl]-N-(2,2-difluorethyl)-4-hydroxy-2-oxo-2,5-dihydrofuran-3-carboxamid in 50 ml Butyronitril werden bei Raumtemperatur 0,5 g Kaliumhydrogensulfat zugesetzt. Die Mischung wird 5 Stunden auf Rückfluß erhitzt. Anschließend wird auf Raumtemperatur abgekühlt und mit 30 ml Wasser gewaschen. Das Lösungsmittel wird im Vakuum entfernt. Man erhält 1 g 4-[[(6-Chlorpyridin-3-yl)methyl](2,2-difluorethyl)amino]furan-2(5H)-on (dies entspricht 77 % Ausbeute).

### Beispiel 2: Herstellung von N-[(6-Chlorpyridin-3-yl)methyl]-N-(2,2-difluorethyl)-4-hydroxy-

### 2-oxo-2,5-dihydrofuran-3-carboxamid

10g Methyl-4-hydroxy-2-oxo-2,5-dihydrofuran-3-carboxylat werden in 111 g Butyronitril vorgelegt und mit 5 g N-[(6-Chlorpyridin-3-yl)methyl]-2,2-difluorethanamin versetzt. Die Lösung wird 3 h auf 65 °C erhitzt. Anschließend wird die Lösung mit 300 ml Wasser extrahiert und anschließend mit 300 ml 5 %iger Salzsäure-Lösung gewaschen. Die organische Phase wird über Magnesiumsulfat getrocknet und das Lösungsmittel im Vakuum entfernt. Zur Reinigung wird aus Isopropanol umkristallisiert.
NMR(CD₃CN): 1H(s, 8,19 ppm); 1H (d, 7,63 ppm ); 1H (d, 7,24 ppm ); 1 H (t, 6,09 ppm); 2 H (s, 4,62 ppm); 2 H (s, 3,98 ppm); 2 H (m, 3,62 ppm)

## Patentansprüche

1. Verfahren zum Herstellen von Verbindungen der Formel (I) 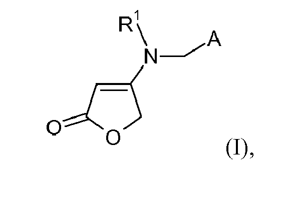 **dadurch gekennzeichnet, dass** man Verbindungen der Formel (II) 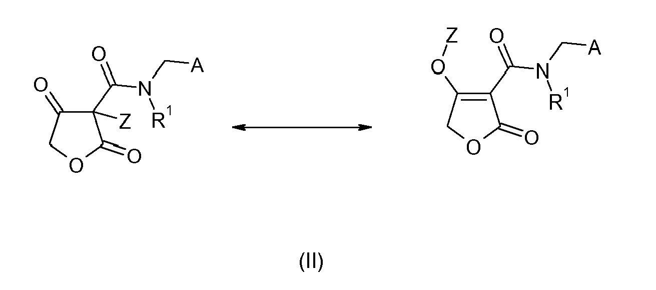 zu Verbindungen der Formel (I) umsetzt, wobei
R¹ für Wasserstoff, Alkyl, Halogenalkyl, Alkenyl, Halogenalkenyl, Alkinyl, Cycloalkyl, Cycloalkylalkyl, Halogencycloalkyl, Alkoxy, Alkyloxyalkyl, Halogencycloalkylalkyl oder Arylalkyl steht und
Z für Wasserstoff, Alkalimetall oder Erdalkalimetall steht und
A für Pyrid-2-yl oder Pyrid-4-yl steht oder für Pyrid-3-yl, welches gegebenenfalls in 6-Position substituiert ist durch Fluor, Chlor, Brom, Methyl, Trifluormethyl oder Trifluormethoxy oder für Pyridazin-3-yl, welches gegebenenfalls in 6-Position substituiert ist durch Chlor oder Methyl oder für Pyrazin-3-yl oder für 2-Chlor-pyrazin-5-yl oder für 1,3-Thiazol-5-yl, welches gegebenenfalls in 2-Position substituiert ist durch Chlor oder Methyl, oder für
für Pyrimidinyl, Pyrazolyl, Thiophenyl, Oxazolyl, Isoxazolyl, 1,2,4-Oxadiazolyl, Isothiazolyl, 1,2,4-Triazolyl oder 1,2,5-Thiadiazolyl, welcher gegebenenfalls durch Fluor, Chlor, Brom, Cyano, Nitro, C₁-C₄-Alkyl (welches gegebenenfalls durch Fluor und/oder Chlor substituiert ist), C₁-C₃-Alkylthio (welches gegebenenfalls durch Fluor und/oder Chlor substituiert ist), oder C₁-C₃-Alkylsulfonyl (welches gegebenenfalls durch Fluor und/oder Chlor substituiert ist), substituiert ist, oder für 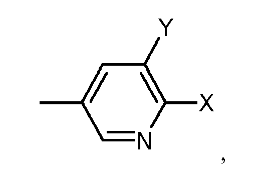 wobei
X für Halogen, Alkyl oder Halogenalkyl steht und
Y für Halogen, Alkyl, Halogenalkyl, Halogenalkoxy, Azido oder Cyan steht.

2. Verfahren nach Anspruch 1, **dadurch gekennzeichnet, dass**
A ausgewählt ist aus der Gruppe bestehend aus 6-Fluor-pyrid-3-yl, 6-Chlor-pyrid-3-yl, 6-Brompyrid-3-yl, 6-Chlor-1,4-pyridazin-3-yl, 2-Chlor-1,3-thiazol-5-yl, 2-Chlor-pyrimidin-5-yl, 5-Fluor-6-chlor-pyrid-3-yl, 5,6-Dichlor-pyrid-3-yl, 5-Brom-6-chlor-pyrid-3-yl, 5-Fluor-6-brom-pyrid-3-yl, 5-Chlor-6-brom-pyrid-3-yl, 5,6-Dibrom-pyrid-3-yl, 5-Methyl-6-chlor-pyrid-3-yl, 5-Chlor-6-iod-pyrid-3-yl und 5-Difluormethyl-6-chlor-pyrid-3-yl und
R¹ ausgewählt ist aus der Gruppe bestehend aus Methyl, Ethyl, Propyl, Vinyl, Allyl, Propargyl, Cyclopropyl, Alkoxyalkyl, 2-Fluor-ethyl, 2,2-Difluor-ethyl und 2-Fluorcyclopropyl, und
Z ausgewählt ist aus der Gruppe bestehend aus Natrium, Kalium und Wasserstoff.

3. Verfahren nach Anspruch 1 oder 2, **dadurch gekennzeichnet, dass**
A ausgewählt ist aus der Gruppe bestehend aus 6-Chlor-pyrid-3-yl, 6-Brom-pyrid-3-yl, 6-Chlor-1,4-pyridazin-3-yl, 2-Chlor-1,3-thiazol-5-yl, 5-Fluor-6-chlor-pyrid-3-yl und 5-Fluor-6-brom-pyrid-3-yl und
R¹ ausgewählt ist aus der Gruppe bestehend aus Methyl, Ethyl, n-Propyl, n-Prop-2-enyl, n-Prop-2-inyl, Cyclopropyl, Methoxyethyl, 2-Fluorethyl und 2,2-Difluor-ethyl und
Z ausgewählt ist aus der Gruppe bestehend aus Natrium und Wasserstoff;

4. Verfahren nach einem der Ansprüche 1 bis 3, **dadurch gekennzeichnet, dass** die Umsetzung in einem Lösungsmittel durchgeführt wird, das ausgewählt ist aus der Gruppe bestehend aus Dioxan, Butyronitril, Propionitril, Acetonitril, DME, Toluol, Methyl-THF, Dichlorbenzol, Chlorbenzol, n-Heptan, iso-Butanol, n-Butanol, Ethanol, Methyl-tert.-butylether, Isopropylethylether und Mischungen davon.

5. Verfahren nach einem der Ansprüche 1 bis 4, **dadurch gekennzeichnet, dass** die Umsetzung in Gegenwart einer Bronsted-Säure durchgeführt wird.

6. Verfahren nach einem der Ansprüche 1 bis 5, **dadurch gekennzeichnet, dass** die Umsetzung in einem Temperaturbereich von 20 °C bis 150 °C durchgeführt wird.

7. Verbindungen der Formel (II) 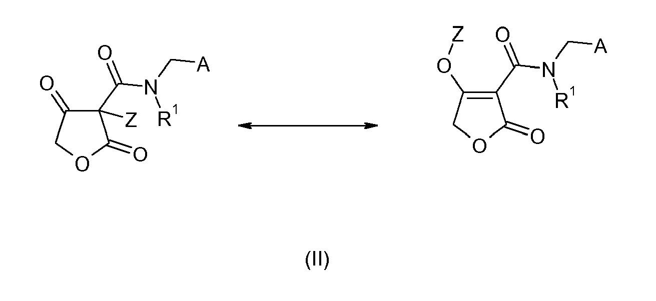 in welcher die Reste A, Z und R¹ wie in einem der Ansprüche 1 bis 3 definiert sind.
